(19) Europäisches Patentamt / European Patent Office / Office européen des brevets

(11) **EP 3 050 943 A1**

(12) **DEMANDE DE BREVET EUROPEEN**

(43) Date de publication:
03.08.2016  Bulletin 2016/31

(21) Numéro de dépôt: **15305091.9**

(22) Date de dépôt: **27.01.2015**

(51) Int Cl.:
*C10L 3/08* (2006.01)    *C10L 5/36* (2006.01)
*C10L 9/08* (2006.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(71) Demandeurs:
• **INSTITUT NATIONAL DE LA RECHERCHE AGRONOMIQUE**
  **75007 Paris (FR)**
• **CENTRE DE COOPERATION INTERNATIONALE EN RECHERCHE AGRONOMIQUE POUR LE DEVELOPPEMENT - CIRAD**
  **75016 Paris (FR)**

(72) Inventeurs:
• **Dumas, Claire**
  **31400 Toulouse (FR)**
• **Barakat, Abdellatif**
  **34070 Montpellier (FR)**
• **Commandre, Jean-Michel**
  **34790 Grabels (FR)**
• **Leboeuf, Alexandre**
  **51210 Le Gault-Soigny (FR)**
• **Rouau, Xavier**
  **34000 Montpellier (FR)**
• **Carrere, Hélène**
  **11120 Saint Marcel sur Aude (FR)**

(74) Mandataire: **Gevers & Orès**
  **36 rue de Saint-Pétersbourg**
  **75008 Paris (FR)**

(54) **Procédé de prétraitement thermo-chimique d'une biomasse ligno-cellulosique en voie sèche**

(57)     Procédé de fractionnement d'une biomasse ligno-cellulosique comprenant une étape de traitement chimique alcalin en voie sèche, une étape de traitement thermique, et une étape de traitement mécanique.

Figure 2

EP 3 050 943 A1

**Description**

La production de bioénergie à partir d'une biomasse ligno-cellulosique (LC) est considérée comme une alternative prometteuse face à l'épuisement des énergies fossiles, en particulier du pétrole. Elle peut être utilisée pour produire par exemple du bioéthanol ou « biofuel » et du biogaz, mais aussi pour produire des biomolécules et synthons pouvant se substituer aux produits issus de la pétrochimie, pour la synthèse de polymères et de biomatériaux tels que des résines ou certains matériaux composites.

La biomasse LC est composée principalement de cellulose, d'hémicellulose et de lignine. Sa nature composite et sa structure matricielle rendent difficiles l'accès des enzymes aux molécules pouvant être transformées par voie biologique ainsi que sa digestion. Il est donc nécessaire de prétraiter la biomasse LC afin d'améliorer l'efficacité de l'hydrolyse enzymatique des polymères pariétaux, étape-clé pour la production de bioéthanol, de méthane et autres biomolécules.

Ainsi un grand nombre de procédés de prétraitement ont été développés (Girio et al., 2010, Biosource Technology, 101 :4775-4800). Ils sont de trois types : mécanique, chimique ou thermique.

Les prétraitements mécaniques, notamment le broyage, permettent d'améliorer l'accessibilité enzymatique et la production de synthons sans générer d'effluents. Toutefois, la quantité d'énergie requise est souvent très élevée ; elle dépend de la nature de la biomasse et du type de broyeur utilisé.

Les prétraitements chimiques sont couramment utilisés pour augmenter l'accessibilité enzymatique de la cellulose et de l'hémicellulose. Ces prétraitements sont réalisés en présence d'un acide, d'une base, d'un oxydant... Les prétraitements acides sont utilisés pour éliminer les hémicelluloses et solubiliser la cellulose. Ils peuvent être menés avec des acides concentrés ou dilués, sachant que l'utilisation d'acides concentrés a pour inconvénient de conduire à la formation de composés inhibiteurs de la fermentation. Les prétraitements alcalins solubilisent principalement la lignine et les hémicelluloses. Ils peuvent être réalisés en présence de solution d'hydroxyde de sodium, de potassium, de calcium ou d'ammonium. Ces traitements chimiques ont l'inconvénient d'utiliser une grande quantité d'eau, de solvants et de réactifs chimiques, tout en générant beaucoup d'effluents toxiques qui doivent ensuite être traités et éliminés.

Les prétraitements thermiques constituent des procédés plus respectueux de l'environnement, comparés aux prétraitements chimiques (Chew et al. 2011 Renewable and Sustainable Energy Reviews, 15 : 4212-4222), mais ils consomment beaucoup d'énergie thermique.

Ces différentes méthodes présentent des avantages et inconvénients : certaines consomment une grande quantité d'énergie, d'autres sont très corrosives et génèrent des produits inhibiteurs vis-à-vis des réactions de biotransformation et des effluents qui nécessitent plusieurs étapes de séparation et de purification très couteuses en énergie et en investissement.

Pour surmonter ces inconvénients, des procédés combinés, par exemple un traitement mécanique couplé à un traitement chimique en voie humide (Barakat et al. 2013 Biosource Technology, 134 : 362-373 ; Van Dyk et al. 2012 Biotechnology Advances 30 :1458-1480) ou à un traitement thermique (Repellin et al. 2010 Biomass and Energy 34(7) : 923-930; Chew et al., 2011 Renewable and Sustainable Energy Reviews, 15: 4212-4222) ont été développés. Plus récemment, un procédé en voie sèche combinant un traitement chimique à la soude suivi d'un traitement mécanique a été décrit (Barakat et al. 2014 Applied Energy 113 : 97-105).

Pour être rentable, un procédé de bioraffinerie doit être développé en tenant compte de deux paramètres majeurs : le coût et l'impact carbone. Les différents procédés développés jusqu'à présent ne répondent pas pleinement à ces critères de rentabilité.

Afin de produire des bioénergies en maitrisant les coûts énergétiques et l'impact environnemental, les inventeurs ont maintenant développé un procédé innovant de prétraitement d'une biomasse LC en voie sèche en combinant des procédés chimique, thermique et mécanique.

RESUME DE L'INVENTION

La présente invention concerne un procédé de fractionnement en voie sèche d'une biomasse LC comprenant une étape de traitement chimique alcalin suivie d'une étape de traitement thermique par torréfaction et d'une étape traitement mécanique par broyage ultrafin. Elle concerne également un procédé de production de bioénergies obtenues par biotransformation d'une biomasse LC prétraitée selon le procédé de fractionnement de l'invention, et plus généralement un procédé de production de produits valorisables. La présente invention a aussi pour objet la biomasse prétraitée en appliquant le procédé de fractionnement de l'invention.

DESCRIPTION DETAILLEE DE L'INVENTION

La présente invention a pour objet un procédé de fractionnement en voie sèche de biomasse LC comprenant une étape de traitement chimique alcalin suivie d'une étape de traitement thermique et d'une étape de traitement mécanique par broyage ultrafin.

Le procédé de fractionnement selon l'invention est donc un procédé de prétraitement combinant trois étapes successives : chimique en voie sèche, thermique et mécanique. Les termes « procédé de fractionnement d'une biomasse » ou « procédé de prétraitement d'une biomasse» sont donc équivalents et seront utilisés indifféremment dans le texte de la présente demande.

Ces étapes de prétraitement sont essentielles car elles vont permettre une hydrolyse très efficace de la cellulose et des hémicelluloses obtenues à partir d'une biomasse LC et une libération accrue des produits valorisables par rapport aux procédés décrits antérieurement.

En particulier, le procédé de fractionnement selon l'invention a l'avantage de nécessiter de faibles quantités d'eau et de produits chimiques ce qui permet de limiter la production d'effluents. Un prétraitement à la soude seule et couplé avec une torréfaction permettent d'améliorer l'accessibilité des holocelluloses. Alternativement, un prétraitement au peroxyde d'oxygène permet d'améliorer l'accessibilité de la cellulose.

Le procédé de fractionnement ou de prétraitement selon l'invention a pour autre avantage de présenter un bilan énergétique positif. La biomasse ainsi prétraitée subit ensuite une transformation biologique qui permet la production de gaz et d'espèces condensables et en particulier de méthane et de bioéthanol.

Comme cela va être exposé ci-après de manière détaillée, ce procédé permet une amélioration quantitative (production accrue d'énergie) et qualitative (production de nombreux produits valorisables).

La biomasse LC qui peut être utilisée dans un procédé selon l'invention comprend les co-produits agricoles et forestiers tels que les pailles, le bois, les tiges et les feuilles. En particulier, les pailles sont une matière première particulièrement adaptée à un tel procédé. L'application du procédé selon l'invention à la paille de blé est présentée à titre d'exemple dans la partie expérimentale.

Le procédé de fractionnement selon l'invention a notamment pour caractéristique d'être réalisé « en voie sèche », notamment l'étape de prétraitement chimique est effectuée en voie sèche.

Par procédé de fractionnement « en voie sèche », on entend un procédé mis en oeuvre en limitant la quantité d'eau ajoutée. La quantité d'eau présente, notamment lors du prétraitement chimique en voie sèche d'une biomasse, est généralement d'environ 30%, et peut être réduit à 20%, voire à 10% (en poids/poids de la biomasse). Ce type de procédé a l'avantage, par rapport aux procédés en voie liquide, de ne nécessiter ni traitement, ni séparation de la phase liquide/solide. Il permet donc de minimiser l'apport en eau, de diminuer le traitement des effluents et de diminuer les coûts d'investissement et de transport ultérieurs de la biomasse prétraitée.

La première étape du procédé de fractionnement d'une biomasse LC selon l'invention est donc un traitement chimique alcalin en voie sèche réalisé par imprégnation d'une biomasse avec la solution alcaline, par exemple à l'aide d'un spray, d'un aérosol ou sous forme de gaz.

L'étape de traitement alcalin peut être réalisée à l'aide de différentes solutions basiques notamment de sodium, de calcium ou de potassium qui peuvent être, par exemple, sous forme de solution d'hydroxyde de calcium, d'hydroxyde de sodium, d'ammoniac, de soude ou de peroxyde d'oxygène. Toute solution basique adaptée à l'utilisation selon l'invention peut être utilisée. Dans un mode préféré de l'invention, la solution basique est une solution de soude (NaOH) ou de peroxyde d'oxygène ($H_2O_2$), de préférence à une concentration comprise environ entre 1% à 20%. Dans un mode de réalisation préférée, la concentration de la solution est comprise entre 5 et 15%, en particulier entre 5% et 10%. Dans un mode de réalisation particulièrement préféré, l'imprégnation de la biomasse est réalisée avec une solution de peroxyde d'oxygène à 10% ou une solution de soude à 10%.

La deuxième étape du procédé de fractionnement d'une biomasse LC selon l'invention est un traitement thermique. Selon un mode préféré de mise en oeuvre, le traitement thermique est réalisé par torréfaction.

De manière générale, la torréfaction consiste à soumettre la biomasse à un traitement thermique à une température comprise entre 100°C et 300°C sans oxygène (en présence de $N_2$) pour dégrader les propriétés mécaniques et modifier la composition chimique de la matrice LC.

Afin d'optimiser la torréfaction (c'est-à-dire diminuer la température et limiter la production des inhibiteurs de la fermentation), la présente invention propose de combiner la torréfaction avec une imprégnation chimique. Cette dernière permet une fragilisation et une modification structurale de l'architecture pariétale pour déstructurer la matière à coeur et augmenter sa réactivité.

Par « torréfaction », on entend, au sens de l'invention, l'exposition de la biomasse à une température modérée comprise entre 100°C et 300°C en absence d'oxygène. Dans le procédé de l'invention, la torréfaction peut être réalisée à des températures comprises entre 100°C et 240°C pendant une durée de 10 min à 120 min. Selon un mode de réalisation préférée de l'invention, la torréfaction peut être réalisée à une température de 140°C, 160°C, 175°C, 190°C ou 200°C. Dans un mode de réalisation particulier, la torréfaction est réalisée à une température modérée allant de 140°C à 200°C pendant une durée de 45 min.

En effet, les inventeurs ont mis en évidence que des températures supérieures à 200°C ont un effet négatif sur le rendement de la méthanisation et de la fermentation ; au-delà de 200°C, la biomasse serait trop dégradée. De plus, l'effet positif du prétraitement thermique de la biomasse est significatif à des températures supérieures à 100°C.

Ainsi, dans un mode de mise en oeuvre préféré, la biomasse est soumise à des températures allant de 100°C à 200°C, de préférence de 140°C à 200°C.

Dans un mode de réalisation particulier de l'invention, la biomasse subit d'abord un traitement chimique alcalin par imprégnation d'une solution de soude à 10% ou de peroxyde d'oxygène à 10% puis une torréfaction entre 140°C et 200°C. Dans un mode de réalisation préféré, le prétraitement de la biomasse consiste en un traitement chimique par imprégnation d'une solution de soude à 10% suivi d'une torréfaction à 140°C. Dans un autre mode de réalisation préféré, le prétraitement de la biomasse consiste en un traitement chimique par imprégnation d'une solution de soude à 10% suivi d'une torréfaction à 200°C.

L'étape de torréfaction présente plusieurs avantages en sus de celui de permettre de fractionner la biomasse par action thermique. En effet, elle permet de récupérer des produits valorisables avant transformation par voie biologique de la biomasse prétraitée. Ces produits, dont certains ont une forte valeur ajoutée, sont exploitables dans d'autres types d'industrie (chimie, cosmétique, pharmacie, alimentaire).

Par « produits valorisables », on entend toute matière exploitable dans tout type d'application dès lors qu'elle présente une utilité, qui est extraite de la biomasse LC lors de l'une des étapes du procédé de prétraitement d'une biomasse ou lors du procédé de production de bioénergies selon l'invention.

Les produits valorisables obtenus grâce à la torréfaction sont constitués de gaz « secs » et d'espèces condensables à température ambiante

Dans un mode préféré de mise en oeuvre selon l'invention, la torréfaction est réalisée dans un réacteur qui permet de recueillir après torréfaction d'une part des espèces condensables à température ambiante et d'autre part des espèces incondensables à température ambiante ou « gaz secs ». Les produits obtenus à la fois sont forme gazeuse, liquide et solide sont ensuite quantifiés. Les produits recueillis lors de la torréfaction ou par condensation suite à la torréfaction font partie de ce que l'on qualifie de « produits valorisables ».

La présente invention a donc pour objet un procédé de production de produits valorisables à partir d'une biomasse LC comprenant la mise en oeuvre du procédé de fractionnement selon l'invention.

Parmi les gaz « secs » majoritaires obtenus se trouvent le dioxyde de carbone ($CO_2$) et le monoxyde de carbone (CO).

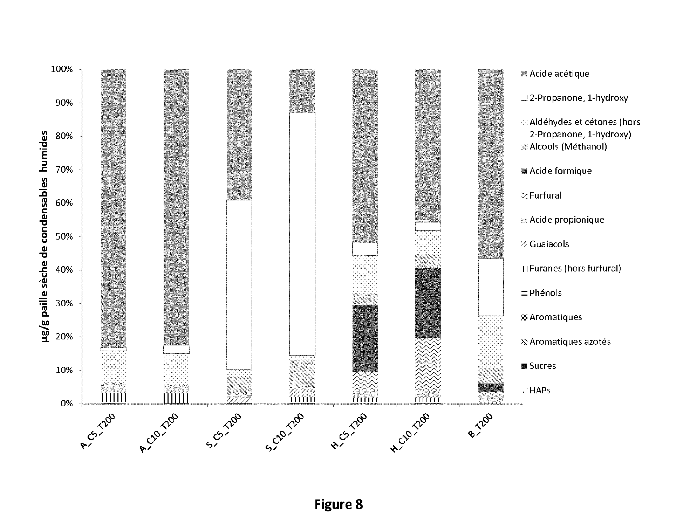

Parmi les espèces condensables obtenues, on peut citer l'acide acétique, le 2-propanone,1-hydroxy, des aldéhydes et des cétones, des alcools dont le méthanol, l'acide formique, le furfural, l'acide propionique, des guaiacols, des furanes, des phénols, des aromatiques et aromatiques azotés, des sucres et des hydrocarbures aromatiques polycycliques (HAPs). La quantité relative de chacune de ces espèces varie en fonction de la nature de la biomasse de départ et des paramètres du procédé, en particulier en fonction du prétraitement chimique (Figure 8).

En effet, une sélectivité des espèces condensables est observée en fonction du prétraitement chimique. Cet effet ressort clairement de la Figure 8. Par exemple, la production de méthanol et de 2-propanone, 1-hydroxy sera fortement favorisée par un prétraitement couplant une imprégnation à la soude à 5% ou de manière plus accentuée si l'on utilise de la soude à 10%, avec une torréfaction à 200°C. De même, la production d'acide acétique, d'acide formique et de furfural seront particulièrement fortes si le procédé de prétraitement comprend une imprégnation au peroxyde d'oxygène (à 5% ou 10%, l'utilisation d'une solution à 10% permettant d'augmenter de plus de 50% la production de furfural) et une torréfaction à 200°C. Les conditions de production d'autres produits d'intérêt sont décrites à la Figure 8. Les conditions opératoires du procédé pourront être déterminées en fonction des espèces chimiques recherchées. Ces espèces seront sélectionnées pour l'intérêt des molécules vertes dans les filières telles que les cosmétiques, la pharmacie ou l'alimentaire, pour leur coût de production comparé à celui de la filière classique, ou pour la pureté des espèces après séparation.

Dans un mode de réalisation particulièrement avantageux, le traitement chimique est couplé à l'étape de torréfaction, ce qui permet d'utiliser l'énergie liée à la montée en température du torréfacteur pour sécher les résidus liquides ou humides résultant du traitement chimique ; ce couplage permet d'optimiser les dépenses énergétiques.

De plus, la torréfaction dégage de l'énergie. La quantité d'énergie produite dépend des échantillons, à savoir de leur nature et du prétraitement qu'ils ont subi, ainsi que des conditions opératoires de la torréfaction. Dans le calcul du bilan énergétique lié à la torréfaction sont intégrés l'énergie nécessaire au chauffage de l'échantillon pour son séchage puis pour sa torréfaction et l'énergie obtenue via la combustion des matières volatiles dégagées (acide acétique, phénols...) durant la torréfaction. Le mode de calcul des énergies liées à ce procédé est détaillé dans la partie expérimentale.

De manière intéressante, lors de la torréfaction, les espèces combustibles gazeuses peuvent être soit récupérées et exploitées en tant que telles, soit brûlées pour produire de l'énergie. A titre d'exemple, le méthane peut être soit brûlé immédiatement lors de l'étape de torréfaction, soit être récupéré et utilisé ultérieurement en tant que biocarburant. Lorsque le bilan énergétique global a été réalisé pour comparer les différentes variantes du procédé, les espèces combustibles ont été brûlées.

Le procédé de fractionnement selon l'invention comprend enfin une étape de traitement mécanique, à savoir une étape de broyage ultrafin, qui fait suite au traitement chimique et à la torréfaction. L'objectif de ce broyage est d'obtenir des poudres ultrafines utilisables dans les transformations biologiques ultérieures.

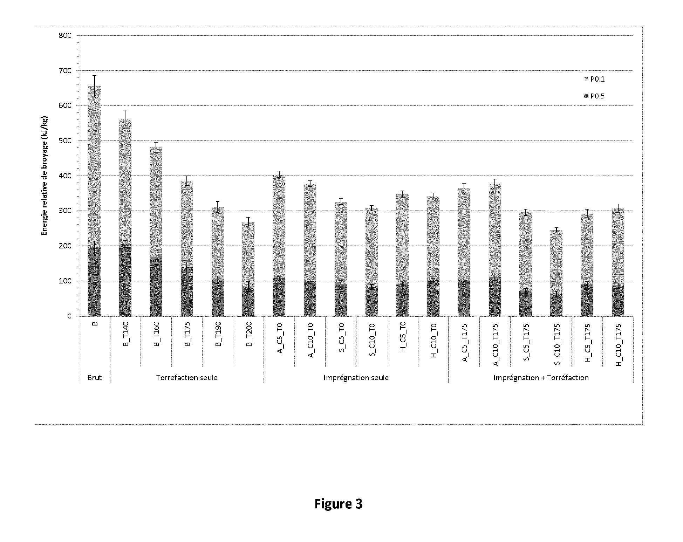

Par « broyage ultrafin », on entend un broyage permettant le passage des particules à travers une grille dont le diamètre est compris entre 0,5 mm et 0,1 mm. Ce broyage peut être réalisé dans tout type de broyeur adapté; les différents types de broyeurs existants sont bien connus de l'homme du métier. Dans un mode de réalisation préféré de l'invention, le broyage ultrafin permet l'obtention de particules pouvant passer à travers une grille de 0,5 mm à 0,1 mm de diamètre. Comme cela ressort de la Figure 3, l'énergie de broyage augmente avec la finesse de la grille.

Il est à noter que, préalablement à la mise en oeuvre du procédé de fractionnement selon l'invention, la biomasse LC subit un pré-broyage. Cette étape a pour but de rendre plus malléable la matière première qui se trouve transformée en une matière relativement homogène. Cette étape de pré-broyage est une étape commune à tous les procédés de prétraitement d'une biomasse LC décrits et expérimentés dans la présente demande. Dans un mode de réalisation de l'invention, les particules obtenues à l'issue de cette étape de prébroyage peuvent passer à travers une grille de 2 mm de diamètre.

Pour résumer, le procédé de fractionnement de l'invention présente de nombreux avantages qui résultent du couplage de trois étapes de prétraitement : tout d'abord un prétraitement chimique en voie sèche, suivi d'une torréfaction à température modérée et enfin un broyage ultrafin. La biomasse LC ainsi prétraitée est un substrat particulièrement adapté à une transformation par voie biologique dont le rendement sera de ce fait nettement amélioré, comme cela va être exposé maintenant.

La présente invention a donc pour autre objet un procédé de production de bioénergies à partir d'une biomasse LC prétraitée par application du procédé de fractionnement selon l'invention, comprenant une étape de transformation par voie biologique de ladite biomasse prétraitée.

Ces bioénergies font partie des produits valorisables au sens de l'invention. De même, les synthons, les biopolymères et autres biomolécules sont des produits valorisables obtenus par le procédé de production de bioénergies de l'invention.

Ainsi, la présente invention a aussi pour objet un procédé de production de synthons et biomolécules qui comprend une étape de prétraitement d'une biomasse LC par application du procédé de fractionnement de l'invention et une étape de transformation biologique de la biomasse ainsi prétraitée.

La présente invention concerne également une méthode de production de produits valorisables obtenus à partir d'une biomasse LC, cette méthode comprenant une étape de prétraitement de ladite biomasse par application du procédé de fractionnement de l'invention. Les produits valorisables peuvent être récupérés directement après la torréfaction. Alternativement, en fonction des produits valorisables d'intérêt, le prétraitement de la biomasse peut être suivi d'une étape de transformation biologique et les produits valorisables récupérés après cette étape de transformation.

La présente invention a donc pour objet une méthode de production de produits valorisables à partir d'une biomasse LC comprenant une étape de prétraitement de ladite biomasse par application du procédé de fractionnement de l'invention suivie, optionnellement, d'une étape de transformation par voie biologique de ladite biomasse obtenue à l'étape précédente, et enfin d'une étape de récupération des produits valorisables obtenus. Les produits valorisables obtenus au cours de ce procédé incluent des gaz, des produits condensables, des bioénergies, des synthons, des biopolymères et autres biomolécules en quantité variable en fonction des conditions opératoires. Par « bioénergies », on entend des biogaz tels que le méthane ($CH_4$) ou le bioéthanol, aussi appelé « biofuel ». Ces bioénergies qui constituent des biocarburants, font partie des produits valorisables au sens de l'invention.

La transformation par voie biologique de la biomasse LC peut être réalisée, par exemple, par méthanisation, par hydrolyse enzymatique ou par fermentation alcoolique.

La méthanisation peut être définie comme une digestion anaérobie qui transforme la matière organique en digestat (possédant des caractéristiques fertilisantes), méthane et gaz carbonique par un écosystème microbien complexe fonctionnant en absence d'oxygène. Le méthane, biogaz majoritairement produit lors de cette réaction, est utilisable comme source d'énergie dans 3 voies principales : énergie thermique, énergie électrique et biocarburant.

La méthanisation peut, par exemple, être réalisée en réacteur grâce à des microorganismes anaérobie provenant par exemple de boues de station d'épuration. Différents protocoles de méthanisation ont été décrits et font partie des connaissances générales de l'homme du métier.

La fermentation alcoolique peut être définie comme un processus biochimique par lequel des carbohydrates, principalement le glucose, sont décomposés en milieu pauvre en oxygène en éthanol et en dioxyde de carbone, avec libération d'énergie. La plupart des microorganismes qui ont la capacité de fermentation alcoolique utilisent cette réaction provisoirement pour gagner de l'énergie, quand l'oxygène nécessaire à la respiration cellulaire manque. Cette réaction permet la production de bioéthanol et de dioxyde de carbone ainsi que des sous-produits tels que des alcools (propanol, isobutanol...) et alcools aromatiques, des esters carboxyliques ou des composés carbonylés. Le bioéthanol, en particulier, est de plus en plus utilisé en tant que carburant.

L'étape de transformation par voie biologique ou biotransformation consiste donc à mettre en contact la biomasse prétraitée avec des enzymes ou des microorganismes capables de dégrader les substrats contenus dans la biomasse.

Afin de démontrer l'amélioration significative en termes de gain énergétique obtenue grâce au procédé de fractionnement selon l'invention, les inventeurs ont réalisé des études comparatives de ce nouveau procédé avec des procédés dans lesquelles la biomasse LC avait subi uniquement un prétraitement thermique ou uniquement un prétraitement chimique avant d'être broyée. La comparaison a été faite à différents niveaux du procédé et les résultats montrent que le procédé de prétraitement combinant à la fois un prétraitement chimique et une torréfaction permet d'obtenir le meilleur bilan quantitatif basé sur le bilan énergétique global du procédé de production de méthane ou de bioéthanol. De plus, l'étape de torréfaction procure un avantage qualitatif du fait qu'elle permet de récupérer des produits valorisables tel que des gaz secs et espèces condensables sans surcoût énergétique spécifique.

Le bilan énergétique des procédés de production de produits valorisables comprenant une étape de prétraitement selon l'invention est particulièrement intéressant.

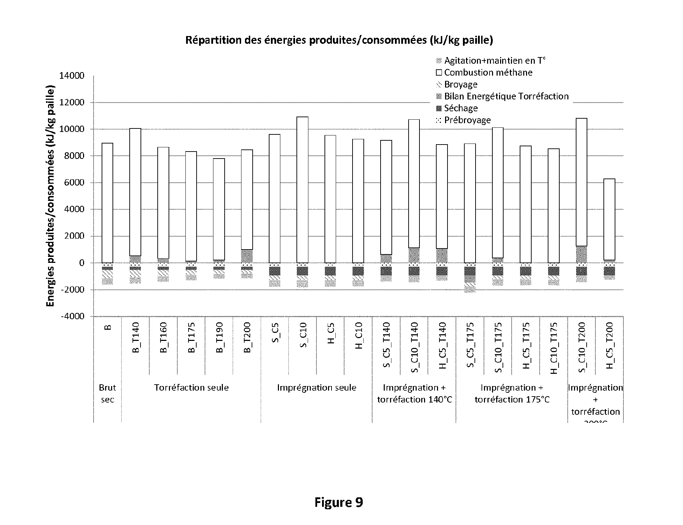

D'un point de vue global, la mise en oeuvre d'un procédé de fractionnement comprenant une étape d'imprégnation chimique alcaline, suivie d'une torréfaction entre 100°C et 240°C et d'un broyage ultrafin permet d'obtenir un bilan énergétique dans lequel la quantité l'énergie produite est très supérieure à la quantité d'énergie consommée. Le bilan énergétique est particulièrement intéressant lorsque l'imprégnation est réalisée avec une solution de soude à 10%, comme cela ressort clairement de la Figure 9. Le bilan énergétique favorable global résulte bien de la diminution de la dépense énergétique associée au prétraitement de la biomasse LC.

Le procédé de fractionnement utilisé pour prétraiter la biomasse LC est également performant d'un point de vue qualitatif du fait des espèces condensables et des gaz « secs » générés lors de l'étape de torréfaction. De plus, une sélectivité des espèces condensables est observée en fonction du prétraitement chimique, comme cela a été précédemment décrit. Il est par conséquent possible de modifier les paramètres du procédé de fractionnement pour favoriser la production de certaines espèces et/ou limiter la production d'autres espèces.

Le procédé de transformation biologique de la biomasse LC prétraitée selon le procédé de fractionnement de la présente invention est également performant si l'on considère le gain de production de bioénergie, que ce soit sous forme de méthane ou de bioéthanol.

La valeur globale du procédé selon l'invention est donc à considérer tant au niveau qualitatif (nature des produits valorisables) que quantitatif (quantité d'énergie produite). A cet égard, le procédé de fractionnement et le procédé de production d'espèces valorisables selon l'invention est très avantageux par rapport à ceux décrits antérieurement. Ainsi, le procédé de prétraitement d'une biomasse LC mis au point par les inventeurs remplit les critères de rentabilité attendus pour un tel procédé en proposant un procédé innovant dans lequel le coût global et l'impact environnemental sont maitrisés.

Des modes de réalisation particuliers sont décrits dans les exemples qui suivent. Ils ont pour seul objectif d'illustrer l'invention et de permettre sa compréhension et ne doivent en aucun cas être considérés comme limitatifs.

DESCRIPTION DES FIGURES

**Figure 1 :** Schéma décrivant l'imprégnation chimique d'une biomasse LC par des solutions basiques de NaOH, $H_2O_2$ et $NH_3$.

**Figure 2 :** Schéma de principe des bilans de matière et d'énergie associés à une procédé de fractionnement d'une biomasse LC comprenant trois étape : (i) imprégnation avec une solution chimique alcaline, (ii) torréfaction et (iii) broyage ultrafin.

**Figure 3 :** Energie de broyage en fonction du prétraitement appliqué. L'énergie de broyage varie en fonction du prétraitement. La partie inférieure de chaque colonne représente l'énergie nécessaire au broyage des substrats organiques de sorte à ce qu'ils passent à travers une grille de 0,5mm (en kJ/Kg de substrat) alors que la partie supérieure de chaque colonne représente l'énergie nécessaire au passage des substrats à travers une grille de 0.1mm (en kJ/Kg de substrat).

**Figure 4 :** Taille de particules ($D_{50}$) en fonction du prétraitement appliqué. Pour chaque condition expérimentale, la colonne de gauche correspond à l'énergie associée à un broyage effectué sur une grille de 0,5mm (d50 en $\mu$m) et la colonne de droite à l'énergie associée à un broyage effectué sur une grille de 0,1mm (d50 en $\mu$m).

**Figure 5 :** Rendement de sucres libérés après hydrolyse enzymatique en fonction du prétraitement appliqué. Pour chaque condition expérimentale, la colonne de gauche correspond à la quantité de glucose produite (en mg/g de substrat) et la colonne de droite à la quantité de sucres réducteurs produite (en mg/g de substrat).

**Figure 6:** Rendement en bioéthanol en fonction du prétraitement appliqué. Chaque colonne représente la quantité d'éthanol produite par fermentation alcoolique (en g/kg de substrat)

**Figure 7 :** Rendement des espèces gazeuses condensables et incondensables en fonction du prétraitement appliqué. La quantité des espèces produites lors de la torréfaction est exprimée en quantités relatives (%).

**Figure 8:** Sélectivité des espèces condensables à 200°C en fonction du prétraitement chimique. La quantité des espèces produites est mesurée en $\mu$g/ g de paille de condensables humides.

**Figure 9 :** Bilan énergétique global. Les énergies produites et consommées selon le type de procédé de prétraitement mis en ouvre (avec ou sans combinaison de prétraitement) sont quantifiées (en kJ/kde paille)

**Figure 10 :** Bilan de la production de méthane. Quantification du méthane disponible à la fin du procédé (kgCH4/kg de paille).

## EXEMPLES

### 1. Matériels et Méthodes

#### 1.1. Substrat LC

Pour assurer la reproductibilité des résultats, toutes les expériences ont été réalisées avec de la paille de blé tendre de la variété Koréli issue d'un même lot.

#### 1.2. Prétraitements

Les prétraitements ont été réalisés selon un même protocole expérimental : les échantillons de paille ont tout d'abord été pré-broyés à l'aide d'un Broyeur Retsch SM100 avec une grille de 2mm, imprégnés, torréfiés et broyés très finement avec un Broyeur Alpine TM UPZ 100 avec deux grilles 0,5mm et 0,1mm.

#### 1.3. Pré-broyage de la paille

Le pré-broyage a été réalisé par une découpe et cisaillement grâce à des couteaux dans un broyeur de type Retsch SM100. La paille a été introduite dans la zone de broyage par une trémie. Le temps de séjour de la paille dans la zone de broyage a été déterminé en fonction de la taille de particules et de la grille choisie. Dès que les particules ont atteint une taille plus fines que la dimension de la grille en sortie du broyeur, à savoir 2mm de diamètre, elles ont été évacuées puis collectées dans le bac de récupération. La vitesse de rotation du rotor a été réglée sur 1390 tr/min. La puissance nominale du moteur dans cette expérience a été de 1500 watts. Un boitier de mesure de la puissance était installé sur l'alimentation du broyeur. Cela a permis, après intégration de la puissance en fonction du temps, de comparer l'énergie de broyage de la paille brute et des pailles traitées.

Ce broyeur a été mis en oeuvre pour le pré-broyage de la paille de blé avec mesure de l'énergie nécessaire à ce pré-broyage.

#### 1.4. Prétraitement chimique

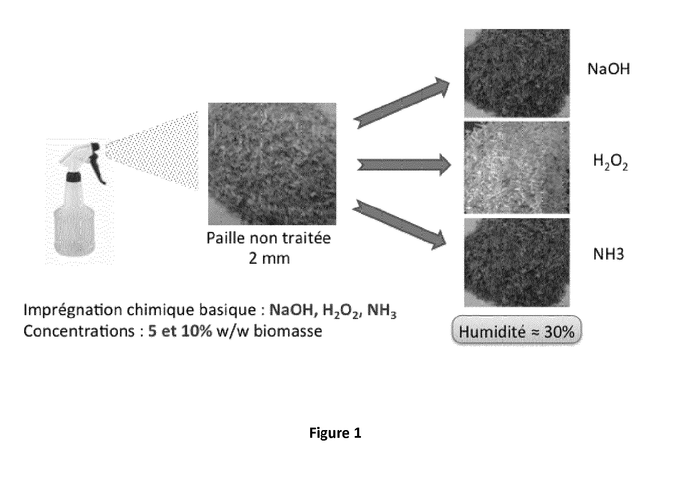

Les traitements chimiques ont été réalisés en voie sèche. Les échantillons ont été imprégnés à l'aide d'un spray par l'ammoniac ($NH_3OH$), le peroxyde d'hydrogène ($H_2O_2$) ou de la soude (NaOH) pour deux concentrations différentes, 5% et 10% w/w (Figure 1). Dans les conditions expérimentales de cette expérience, la torréfaction est réalisée sur un site différent de celui où les échantillons sont traités chimiquement ; de ce fait ces derniers sont séchés à 40°C pour stopper la réaction chimique avant transport.

#### 1.5. Prétraitement thermique par torréfaction

La torréfaction de la paille a été effectuée dans un réacteur TORPILLE. Cinq températures de torréfaction comprises entre 140°C et 200°C ont été mises en oeuvre (140°C, 160°C, 175°C, 190°C et 200°C). Les échantillons traités chimiquement ont été torréfiés à 3 températures (140°C, 175°C et 200°C). Les différentes combinaisons sont reportées dans le Tableau 1.

Ce réacteur repose sur un système batch à lit fixe chauffé dans un four à convection forcée régulé en température. On nommera ce réacteur TORPILLE pour TORréfaction PILote en LaboratoirE. Du diazote ($N_2$) de balayage a

été choisi car il est inerte ; ce gaz a été préchauffé dans un serpentin placé dans le four. Les gaz produits et les espèces condensables ont été maintenus en température en sortie du réacteur pour éviter la condensation. Les espèces condensables ont par la suite été condensées dans 2 serpentins en verre plongés dans un bain thermostaté à -15 °C. Les gaz « secs » ont ensuite été analysés dans une micro-GC Varian, qui permet de quantifier $CO_2$, CO, $CH_4$ et $H_2$. Pour suivre l'évolution de la température, trois thermocouples ont été installés. Deux se situent à l'intérieur du réacteur, en dessous et au-dessus du lit de paille ; le troisième est placé dans le four.

La mesure de la puissance du four a été réalisée en continu grâce à une centrale de mesure Janitza UMG 96 RM. Un débit d'azote de 6 Nl/min a été retenu pour assurer une bonne homogénéisation du lit de paille. Le temps de séjour de 45 minutes a débuté lorsque le thermocouple T3 a atteint la température de consigne. Les condensables récupérés ont été analysés par la suite, tout d'abord avec la méthode Karl Fisher pour déterminer leur teneur en eau, puis par GC-MS pour caractériser et quantifier les principales espèces produites.

**Tableau 1 :** Présentation des différents prétraitements appliqués à la paille de blé et leur nom selon la nomenclature utilisée

| | | Sans torréfaction | 140°C | 160°C | 175°C | 190°C | 200°C |
|---|---|---|---|---|---|---|---|
| **Brut** | | B | B_T140 | B_T160 | B_T175 | B_T190 | B_T200 |
| **Ammoniac NH₃OH** | 5% w/w | A_C5 | A_C5_T140 | Ø | A_C5_T175 | Ø | A_C5_T200 |
| | 10% | A_C10 | A_C10_T140 | Ø | A_C10_T175 | Ø | A_C10_T200 |
| **Peroxyde d'Hydrogène H₂O₂** | 5% | H_C5 | H_C5_T140 | Ø | H_C5_T175 | Ø | H_C5_T200 |
| | 10% | H_C10 | H_C10_T140 | Ø | H_C10_T175 | Ø | H_C10_T200 |
| **Soude NaOH** | 5% | S_C5 | S_C5_T140 | Ø | S_C5_T175 | Ø | S_C5_T200 |
| | 10% | S_C10 | S_C10_T140 | Ø | S_C10_T175 | Ø | S_C10_T200 |

1.6. Broyage ultrafin de la paille

La station de broyage Alpine TM UPZ 100 a été utilisée afin de réduire la taille des particules et obtenir des poudres ultrafines. Elle est constituée d'un axe mobile sur lequel sont fixées des palettes qui projettent le produit contre une grille fixe. Le produit est resté dans la chambre de broyage jusqu'à ce qu'il soit broyé. Il a subi un broyage à la fois par impact et par cisaillement (il a été projeté contre la grille et a circulé contre cette dernière tant qu'il n'est pas passé au travers). La vitesse de rotor a été réglée à 18000 rpm avec une alimentation en continu à 1kg/h. Le broyeur a été équipé d'un wattmètre permettant de mesurer la puissance consommée. Deux grilles 0,5mm et 0,1mm ont été utilisées.

1.7. Production d'espèces consommables ou bioénergies

1.7.1. Potentiel Méthane (BMP)

1.7.1. a. En batch

Le potentiel méthane (BMP ou Biochemical methane potential) correspond au volume maximum de méthane produit lors de la dégradation anaérobie du substrat organique. Il est exprimé en $mLCH4.(g_{MV})^{-1}$ dans les Conditions Normales de Température et de Pression (CNTP : 0°C, 1013 hPa). Le BMP doit donc être considéré comme un outil de caractérisation pour l'évaluation de la biodégradabilité du substrat considéré, et non comme une prédiction de la production en méthane dans le réacteur.

Le test est basé sur la mesure de la production de méthane dans une fiole de 500mL fermée hermétiquement dans laquelle ont été mis en contact une quantité connue d'échantillon à tester (0,999g de MV/fiole, MV : matière volatile, correspond à la matière organique) et des microorganismes anaérobies provenant de boues granulaires traitant des effluents d'une sucrerie de Marseille (64,5mL soit 2g de MV/fiole). Le ratio S/X, c'est-à-dire le rapport quantité de substrat sur quantité de biomasse microbienne était donc de 0,5. Afin d'assurer une bonne activité microbienne, ces derniers ont été placés dans des conditions optimales, par ajout d'une solution de macroéléments, d'une solution d'oligoéléments et d'un tampon bicarbonate pour fixer le pH entre 6,5 et 7,5. Le volume est complété à 400mL avec de l'eau osmosée. L'atmosphère des fioles a été remplacée par de l'azote puis mise en agitation dans une chambre à 37°C afin d'être en condition mésophile anaérobie. Pour chaque échantillon, le test a été réalisé en duplicat et a été poursuivi jusqu'à l'obtention d'un plateau sur la courbe de production de méthane (ml de CH4/$g_{MV}$).

1.7.1.b. En bioréacteurs

Les réacteurs permettent d'évaluer la cinétique de la réaction de biodégradation des échantillons de paille lors la digestion anaérobie. Ces tests permettent également d'évaluer la dégradation des échantillons avec des microorganismes qui ont potentiellement développés des activités propres/spécifiques à la dégradation de ces échantillons grâce à une adaptation de la population microbienne au cours des ajouts successifs de substrat.

Les essais ont été effectués dans quatre réacteurs en verre équipés d'une double enveloppe dans laquelle circule de l'eau chauffée à 37°C. La contenance de chaque réacteur était de 3L (remplis à 2,5L) et l'agitation a été réalisée à l'aide d'un agitateur magnétique. Un compteur de gaz à impulsion (RITTER ® MGC-1 V3), branché en sortie de réacteur, a été utilisé pour mesurer la production de biogaz en continu et l'acquisition des données a été réalisée par le logiciel « ODIN », développé par l'INRA et l'INRIA, toutes les 3 minutes. De même une sonde pH a été plongée dans chaque réacteur, l'acquisition des données s'est faite cette fois avec le logiciel « ICinac ». Toutes les données ont ensuite été mises en ligne sur SILEX (Système d'Information pour l'Expérimentation). Tous les réacteurs ont été initialement inoculés avec les boues granulaires à 60$g_{MS}$/L, provenant d'un procédé UASB (Upflow Anaerobic Sludge Blanket / lits de boue anaérobie à flux ascendant) traitant les effluents de sucrerie. Ils ont été mis sous agitation pendant quelques jours afin de libérer les microorganismes concentrés dans les granules de boue. Dans chaque réacteur, des ajouts successifs d'éthanol (à 0,5gDCO/L puis à 1gDCO/L) ont été réalisés afin de tester l'activité des boues et de mesurer la respiration endogène des boues en fin de réaction.

Suite à cela, des batchs successifs avec une charge de 1 gMV paille/L puis 1,5 gMV paille/L ont été faits avec les échantillons de paille de blé. La masse de ces échantillons a été choisie en fonction des résultats obtenus lors des tests BMP.

1.7.2. Hydrolyse enzymatique et fermentation éthanolique

17.2.a. Hydrolyse enzymatique

L'hydrolyse enzymatique a été réalisée avec un cocktail commercial de cellulase avec 20U FP/g substrat. 100 mg de paille traitée et non traitée ont été mis en suspension dans un tube en verre dans un volume total de 5 ml d'un tampon d'acétate de Na 50 mM à pH=5, dans lequel on a ajouté l'acide de sodium à 3%. Les tubes ont été agités pendant 72h à l'aide d'un agitateur placé dans une étuve à 37°C. À la fin de la réaction, on a prélevé 1 ml dans un eppendorf qui a été porté à 100°C dans un bain marie afin de bloquer la réaction enzymatique. Les échantillons ont ensuite été centrifugés pendant 10 min à 1200 rpm. Le surnageant a été récupéré puis injecté en HPLC pour déterminer la quantité de sucres libérés par les enzymes.

Les conditions opératoires de l'analyse par HPLC :

- Température : 40°C
- Débit : 0,3ml/min
- Volume injecté : 20µl
- Eluant : $H_2SO_4$ 0,0005M
- Colonne : BioRad HPX 87H
- Détecteur: Refractomètre RI

1.7.2.b. Fermentation éthanolique SFS (Saccharification fermentation simultanées)

Après optimisation, la méthode retenue pour l'évaluation du potentiel en fermentation alcoolique des échantillons a consisté à réaliser simultanément la saccharification et la fermentation dans des conditions permettant l'analyse d'un grand nombre d'échantillons en parallèle et en faibles quantités (75mg). Les levures utilisées sont des levures sèches actives (Lallemand), préparées sous forme d'une suspension à 9g/l dans du milieu YNB (Difco) (voir annexe) tamponné par une solution phtalate de potassium 50mM, pH 5,5 (voir annexe). Le cocktail enzymatique a été dilué dans le même tampon pour atteindre une dilution finale de 1/250 pour 300mg de paille. Pour chaque échantillon à tester, une masse d'environ 75mg, mesurée précisément, a été placée dans une fiole à septum pour passeur de chromatographie préalablement stérilisée. Sous hotte à flux laminaire, ont ensuite été ajoutés 0,9ml de suspension de LSA puis 0,1ml de cocktail enzymatique dilué selon le protocole suivant:

- Mise en suspension de 8,1mg de levures sèches dans 0,9ml d'eau stérile.

- Mélange au vortex puis centrifugation à 5000rpm pendant 5minutes à 19°C et élimination du surnagent (x2 dans le même volume d'eau).

- Mise en suspension des levures dans 0.9ml de solution d'YNB et tampon phtalate stérilisé à pH=5, 5.

- Après fermeture du flacon, une aiguille stérile a ensuite été placée à travers le septum pour permettre l'échappement du CO2 libéré lors de la fermentation tout en limitant les pertes d'eau par évaporation.

Après pesée initiale, les fioles ont été ensuite incubées à 28°C. La fermentation a été suivie par pesée des fioles au cours du temps et la différence de perte de masse entre les échantillons et les fioles témoins sans paille a été déterminée. La quantité d'éthanol produite a été calculée à partir des rendements de fermentation en appliquant un facteur 0,47/0,45 à la perte de masse mesurée.

1.8. Bilans matières et énergétiques

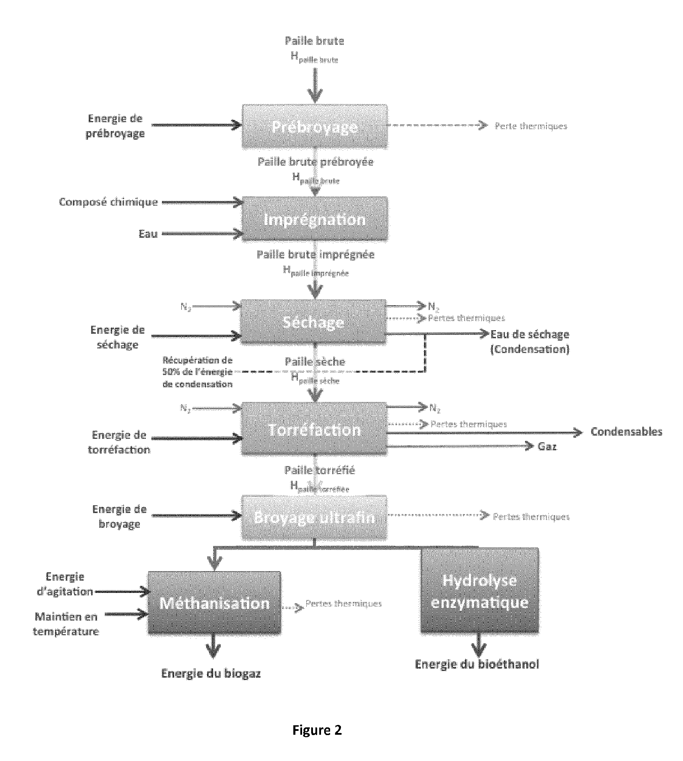

Pour le bilan énergétique et le bilan matière, tous les calculs et les mesures ont été rapportés à un kilogramme de paille sèche brute. Le schéma de la Figure 2 représente les différentes étapes du procédé. La filière la plus complète avec imprégnation et torréfaction de la paille lors du prétraitement a été choisie.

Avant de définir les différents calculs d'énergie, les données initiales suivantes ont été posées:

✓ On suppose que la paille, après le pré-broyage, a une humidité de 10%
✓ L'humidité de la paille imprégnée est de 30%
✓ On suppose que les produits de torréfaction sont valorisés pour produire de la chaleur (combustion)
✓ On suppose que $NH_3$ et $H_2O_2$ sont évaporés pendant le séchage
✓ L'énergie de chaque opération est rapportée à 1kg de paille sèche
✓ On suppose que la réaction de torréfaction est athermique ($\Delta Hr=0$)
✓ Les pertes thermiques sont négligées

1.8.1. Énergie de broyage

Afin de mesurer l'énergie consommée par le broyeur, un wattmètre PX120 (METRIX) relié à un logiciel informatique d'analyse en continu a été utilisé. Deux puissances ont été mesurées: puissance apparente et puissance active. La puissance utile notée P a pour expression P=UI (U : tension en volt et I Intensité en Ampère) P s'exprime en Watt, elle présente la puissance fournie par le distributeur pour alimenter l'ensemble des récepteurs. Afin de calculer l'énergie de broyage, la puissance active (P), la masse récupérée (M), le temps de broyage (3min) ont été relevées. L'énergie broyage se calcule par :

$$\text{Puissance de broyage} * \text{temps} = \text{Energie (Wh)}$$
$$\text{Energie broyage KWh/t} = \text{Energie (Wh)} / (M/1000)$$

1.8.2. Energie de séchage

Il a été supposé que l'énergie de séchage $E_s$ correspond à l'énergie qu'il faut pour élever la température de 20°C à 105°C de la paille et à l'énergie de vaporisation de l'eau. Les pertes thermiques ont été négligées. L'énergie de séchage correspond donc à :

$$E_s = E_{s\_paille} + E_{eau} + E_{evap\_eau}$$

$$E_s = m_{paille_{anhydre}} \cdot Cp_{paille} \cdot (T^{105°C} - T^{\infty}) + m_{eau} \cdot Cp_{eau} \cdot (T^{105°C} - T^{\infty}) + m_{eau} \cdot Lv_{eau\ à\ 105°C}$$

Avec:

- $T^\infty$, température ambiante (°C)
- $Lv_{eau}$, chaleur de vaporisation de l'eau $(kJ.kg^{-1})^{-1}$
- $Cp_{paille}$, chaleur massique de la paille de blé $(kJ.kg^{-1}.K^{-1})$

1.8.3. Energie de torréfaction

Il a donc été supposé que l'énergie nécessaire à la torréfaction Et correspond à la chaleur sensible qu'il faut pour élever la température de 105°C à la température de torréfaction.

$$E_t = m_{paille_{anhydre}}.Cp_{paille}.\left(T^{torréfaction} - T^{105°C}\right)$$

Il a ensuite été décidé de prendre en compte la teneur énergétique de la paille avant et après torréfaction. Pour cela, il a fallu déterminer l'enthalpie de la paille. L'enthalpie standard de formation d'après la loi de Hess a donc tout d'abord été déterminée :

$$\Delta_r H^0_{298} = \Sigma\ \vartheta_i h^0_f\ (i)$$

Les valeurs des hf0 de l'eau et des gaz légers sont aisément accessibles dans les tables des grandeurs thermodynamiques des corps purs. En revanche, elles ne sont pas définies pour des composés complexes comme la biomasse. Il a donc été choisi de se baser sur la réaction de combustion de la paille. Les analyses ultimes de la paille ont servi de point de départ pour définir les compositions molaires. Les formules brutes de la paille ont été exprimées en les ramenant à une base en $C_6$.

La combustion stoechiométrique d'un composé hydrocarboné $C_xH_yO_z$ s'écrit :

$$C_xH_yO_z + n\ O_2 = x\ CO_2 + \frac{y}{2}\ H_2O$$

L'enthalpie standard de cette réaction, aussi appelée chaleur de combustion, est l'opposé du Pouvoir Calorifique Supérieur (PCS en kJ/mol) du réactif.

$$\Delta_r H^0_{298} = -PCS$$

Ce qui permet de déterminer $h_f^0(C_xH_yO_z)$,

$$h_f^0\left(C_xH_yO_z\right) = x.h_f^0(CO_2) + \frac{y}{2}.h_f^0(H_2O) + PCS(C_xH_yO_z)$$

Ainsi pour chaque opération de torréfaction, la valeur de l'enthalpie de formation en entrée et en sortie du réacteur a été déterminée.

L'enthalpie de la paille, en fonction de la température de séchage (105°C) en entrée et en fonction de la température de torréfaction (140°C à 200°C) en sortie a donc été déterminée :

$$H_T = \%_{perte\ de\ masse}\cdot\left(h_f^0\left(C_xH_yO_z\right) + \int_{298}^{T} Cp_{paille}\cdot dT\right)$$

Ce résultat a été utilisé pour calculer l'énergie nécessaire à la torréfaction par la différence des enthalpies en sortie et en entrée :

$$\Delta H_{torréfaction} = H_{T_{sortie}} - H_{T_{entrée}}$$

Le bilan énergétique de la torréfaction a été obtenu et est défini par :

$$E_{Torréfaction} = E_t - m_{gaz}\cdot PCI_{gaz} + m_{condensables\ secs}\cdot PCI_{condensables} + \Delta H_{torréfaction}$$

1.8.4. Energie de fonctionnement pour la méthanisation

Les mesures effectuées sur place pour l'énergie d'agitation et la chaleur nécessaire à la méthanisation en laboratoire se sont avérées très importantes. Il a donc été supposé ici que l'énergie consommée pour la méthanisation à l'échelle laboratoire est surdimensionnée par rapport à une consommation dans un procédé à grande échelle. Pour prendre en compte cette énergie, des données de constructeur de méthaniseur ont permis de quantifier l'énergie nécessaire à l'agitation et au maintien de la température au sein du méthaniseur. L'énergie nécessaire à la méthanisation est définie comme :

$$E_{méthanisation} = 0{,}05\cdot E_{biogaz}$$

1.8.5. Optimisation du procédé

Pour diminuer l'impact énergétique du séchage, l'hypothèse selon laquelle que 50% de l'énergie d'évaporation de l'eau, récupérée par condensation après le séchage, serait recyclée pour l'opération de séchage a été avancée. Ainsi, l'énergie de séchage $E_S$ s'écrit maintenant,

$$E_S = E_s - \frac{1}{2}\ m_{eau}\cdot Lv_{eau\ à\ 105°C}$$

$$E_S = m_{paille_{anhydre}}\cdot Cp_{paille}\cdot\left(T^{105°C} - T^{\infty}\right) + m_{eau}\cdot Cp_{eau}\cdot\left(T^{105°C} - T^{\infty}\right) + \frac{1}{2}\cdot m_{eau}\cdot Lv_{eau\ à\ 105°C}$$

1.8.6. Bilan final du procédé global

En prenant en compte les différents calculs effectués précédemment, les énergies de pré-broyage et de broyage ultrafin, il est possible de faire le bilan énergétique global du procédé ΔE par une voie de valorisation en Biogaz et en Bioéthanol. On notera $E_{biogaz}$ et $E_{bioethanol}$ l'énergie produite par les procédés de méthanisation et de production d'éthanol.

$$\Delta E_{méthanisation} = E_{pré-broyage} + E_S + E_T + E_{broyage} + E_{méthanisation} + E_{biogaz}$$

2. Résultats

2.1. Effet des prétraitements sur l'énergie de broyage et la taille de particules

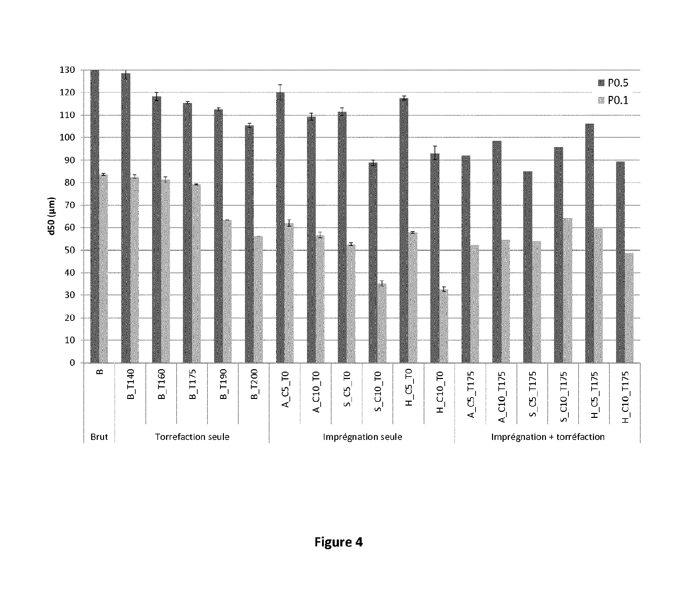

L'effet des prétraitements sur l'énergie de broyage est présenté à la Figure 3 et celui sur la taille de particules est représenté à la Figure 4.

La torréfaction a un effet significatif sur la diminution de l'énergie de broyage (réduction de 60% à 200°C) ainsi que sur la diminution de la taille des particules (réduction de 33% à 200°C). Le prétraitement des échantillons par imprégnations chimiques permet également de réduire considérablement l'énergie de broyage (réduction de 51% avec 10%w/w de NaOH ; réduction de 48% avec 10%w/w de $H_2O_2$) et la taille des particules (réduction de 57% avec 10%w/w de NaOH; réduction de 61% avec 10% w/w de $H_2O_2$). Les prétraitements à la soude se révèlent être plus efficaces que ceux effectués avec $H_2O_2$. Toutefois le couplage de la torréfaction et des prétraitements chimiques ne permet pas d'augmenter significativement ces effets (pas d'effet synergique ou additif évident).

2.2. Effet des prétraitements sur la libération des sucres réducteurs et du glucose et sur fermentation alcoolique

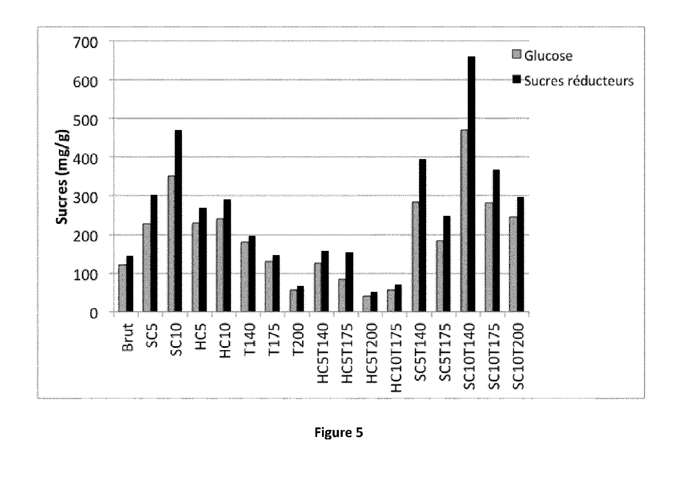

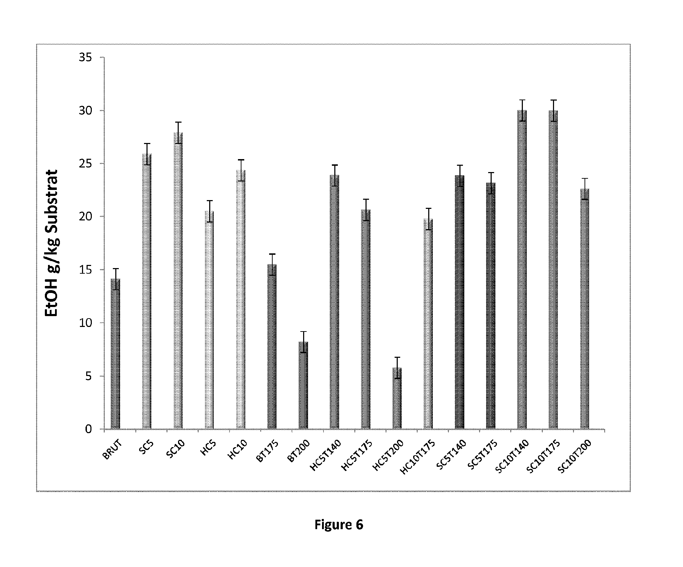

L'effet des prétraitements sur la libération des sucres réducteurs et du glucose après hydrolyse enzymatique est présenté à la Figure 5. L'effet des prétraitements sur les rendements de production de bioéthanol après fermentation alcoolique est présenté à la Figure 6.

La torréfaction se révèle avoir un effet négatif sur la libération des sucres (-54% à 200°C) ainsi que sur la production d'éthanol mais uniquement pour des températures supérieures à 175°C (-42% à 200°C). Les imprégnations chimiques permettent d'augmenter considérablement la libération des sucres (+191% avec NaOH à 10%) et la production d'éthanol (+98% avec NaOH à 10%; +72% avec $H_2O_2$ à 10%). Les prétraitements à la soude se révèlent être plus efficaces que ceux effectués avec $H_2O_2$. Lorsque les imprégnations chimiques sont couplées à la torréfaction, les effets sur la libération de sucres et la fermentation dépendent de l'agent chimique utilisé. Lorsque la soude est couplée à la torréfaction (jusqu'à 175°C), cela a un effet positif sur la libération de sucres (+290% pour SC10-T140) et la fermentation (+116%) alors que lorsque $H_2O_2$ est utilisé en combinaison avec la torréfaction, l'effet positif de l'agent chimique est contrebalancé par l'effet négatif de la torréfaction, dès 140°C sur la libération des sucres (-5%) et à 175°C sur la fermentation (-45% HC5-T175 puis -55% pour HC5-T200).

2.3. Effet des prétraitements sur la production du méthane

La quantité de méthane produite selon différentes conditions expérimentales de prétraitement est présentée au Tableau 2.

**Tableau 2** : Potentiel méthane en fonction de prétraitements

| Echantillon | Potentiel méthane moyen en réacteur (mlCH4/gMV) | Elément de comparaison : Potentiel méthane BMP (mlCH4/gMV) |
|---|---|---|
| **Brut** | **326** | **267± 1** |
| H-C5 | 310 | 284± 14 |
| S-C10 | 377 | 327±3 |
| S-C10-T140 | 376 | 288± 10 |

Les potentiels méthane des échantillons traités à la soude à 10% (S-C10) se révèlent les plus élevés (327mLCH4/gMV). Lorsque les échantillons sont également torréfiés, le potentiel méthane est de l'ordre de 288mLCH4/gMV en batch et 376mLCH4/gMV en bioréacteur. Toutefois, les échantillons à la fois imprégnés chimiquement et torréfiés possèdent un potentiel méthane toujours supérieur à celui de l'échantillon brut. Les échantillons imprégnés avec $H_2O_2$ possèdent des potentiels méthane BMP plus élevés que celui de l'échantillon brut mais en moindre proportion que ceux traités à la soude. Ceci ne se vérifie pas en bioréacteur où les rendements sont similaires. Ainsi le couplage traitement chimique à l'$H_2O_2$ avec la torréfaction qui fait diminuer ce potentiel paraît donc inintéressant.

2.4. Effet des prétraitements sur la production des molécules d'intérêt ou les espèces condensables

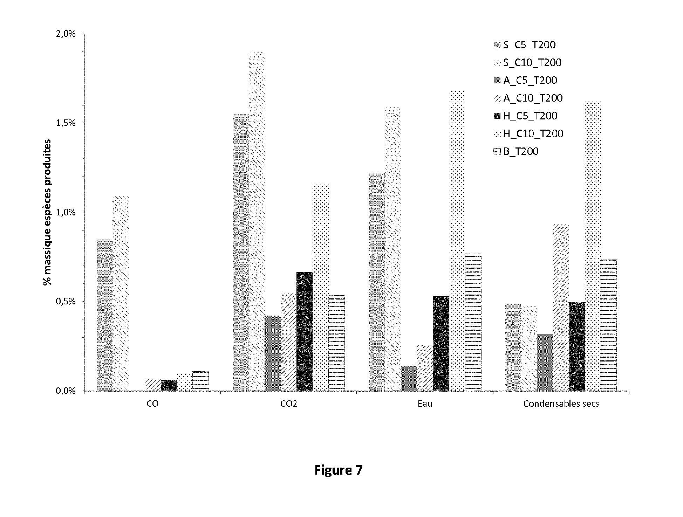

La figure 7 présente les rendements obtenus pour les espèces gazeuses condensables et incondensables en fonction des prétraitements. Il ressort que la soude a une forte influence sur la production de gaz et d'eau (environ 3 fois plus que pour la torréfaction seule). On peut également remarquer une influence de $H_2O_2$ principalement pour les condensables secs et l'eau. Le $NH_3$ n'a que très peu d'influence sur la formation des coproduits avec même une diminution de la quantité de condensables et d'eau.

Concernant l'analyse des condensables présentée sur la figure 8 à 200°C, on constate que l'ammoniac favorise fortement la formation d'acide acétique et de furanes. L'$H_2O_2$ favorise la production de condensables secs comparé à la torréfaction seule dans des proportions équivalentes pour toutes les espèces produites, excepté pour l'acide formique et le furfural qui est produit en quantité très supérieure à la torréfaction classique. Concernant le prétraitement à la soude, on peut voir que la proportion d'acide acétique est beaucoup plus faible et que le composé majoritaire des condensables sec est le 2-propanone,1-hydroxy qui augmente avec la concentration en soude.

De façon générale, on peut remarquer que la température seule a très peu d'influence sur la paille de blé torréfiée, excepté pour 200°C. Cela se confirme avec la quantité de gaz et de condensables produits et sur les analyses physico-chimiques. Les gaz produits sont presque exclusivement composés de $CO_2$. Les condensables sont composés d'eau et de composés organiques en proportions équivalentes. Les composés organiques sont eux principalement composés d'acide acétique, formique et propénoïque, d'aldéhydes, de cétones, de méthanol et de furfural.

Pour le couplage imprégnations chimiques-torréfaction, les résultats sont intéressants avec une augmentation de la perte de masse à 175°C et 200°C avec la soude et le peroxyde d'hydrogène (résultats non montrés). Une sélectivité des espèces condensables est observée en fonction du prétraitement chimique. En effet, le traitement à la soude libère des condensables riches en acide acétique (torréfaction à 140°C ; résultat non montré) alors que $H_2O_2$ permet de libérer des espèces avec des teneurs élevées en phénols - et ce quelle que soit la température de torréfaction (140, 175 ou 200°C) - montrant ainsi l'attaque plus spécifique de la lignine par $H_2O_2$. Pour conclure, les différentes familles d'espèces produites (gaz, eau et composés organiques) représentent chacune un tiers de la perte de masse de la paille torréfiée avec un bouclage du bilan global massique compris entre 96% et 99%.

2.5. Bilan énergétique

Le bilan énergétique effectué sur un procédé de production de méthane à partir de paille de blé est présenté sur la Figure 9. Ce bilan permet de mettre en évidence que les consommations énergétiques sont imputables:

- au pré-broyage et au broyage ;

- au séchage, principalement lorsque l'échantillon a été imprégné par un agent chimique. Il existe toutefois une possibilité d'intégrer la partie séchage dans l'étape de torréfaction pour améliorer le bilan, ce qui revient à torréfier le solide sans passer par une étape de séchage ;

- à l'agitation et au maintien en température du méthaniseur (consommation estimée à 5% du méthane produit par le digesteur).

- à la torréfaction. Toutefois, cette torréfaction peut aussi dégager de l'énergie. Dans ce calcul sont intégrés : l'énergie nécessaire pour chauffer l'échantillon de la température de séchage à celle de torréfaction, l'énergie pouvant être obtenue via la combustion des matières volatiles dégagées (acide acétique, phénols etc...) durant la torréfaction.

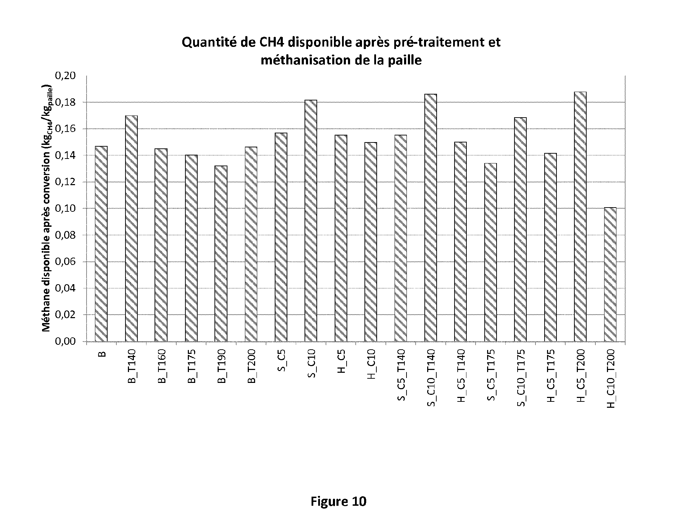

Comme cela ressort de la Figure 10, les prétraitements comprenant une imprégnation à la soude à 10% suivie d'une torréfaction entre 140°C et 200°C et d'un broyage ultrafin sont ceux qui permettent de produire les quantités de CH4 les plus élevées.

**Revendications**

1. Procédé de fractionnement d'une biomasse ligno-cellulosique **caractérisé en ce qu'**il comprend :

   a. une étape de traitement chimique alcalin en voie sèche,
   b. une étape de traitement thermique, et
   c. une étape de traitement mécanique.

**2.** Procédé selon la revendication 1 dans lequel le traitement chimique alcalin est réalisé par imprégnation d'une solution de soude ou d'une solution de peroxyde d'oxygène.

**3.** Procédé selon la revendication 2 dans lequel l'imprégnation est réalisée avec une solution dont la concentration est comprise entre 5% et 10%, de préférence égale à 10%.

**4.** Procédé selon l'une des revendications 1 à 3 dans lequel l'étape de traitement thermique est réalisée par torréfaction à une température comprise entre 100°C et 240°C, de préférence entre 140°C et 200°C.

**5.** Procédé selon l'une des revendications précédentes dans lequel l'étape de traitement mécanique est réalisée par broyage ultrafin.

**6.** Procédé selon l'une des revendications précédentes comprenant en outre, préalablement à l'étape a., une étape de pré-broyage.

**7.** Procédé selon l'une des revendications précédentes dans lequel la biomasse prétraitée est une paille.

**8.** Procédé selon l'une des revendications précédentes dans lequel le procédé de prétraitement est réalisé par imprégnation de la biomasse avec une solution de soude à 10% suivie d'une torréfaction à une température comprise entre 140°C et 200°C.

**9.** Procédé selon l'une des revendications précédentes dans lequel le procédé de prétraitement est réalisé par imprégnation de la biomasse avec une solution de peroxyde d'oxygène à 10% suivie d'une torréfaction à une température comprise entre 140°C et 200°C.

**10.** Biomasse ligno-cellulosique prétraitée obtenue par le procédé selon l'une des revendications 1 à 9.

**11.** Procédé de production de produits valorisables à partir d'une biomasse ligno-cellulosique, **caractérisé en ce qu'**il comprend la mise en oeuvre du procédé selon l'une des revendications 8 ou 9.

**12.** Procédé de production de bioénergie comprenant une étape de transformation, par voie biologique, d'une biomasse prétraitée selon la revendication 10.

**13.** Procédé selon la revendication 12 dans lequel la transformation biologique est réalisée par fermentation alcoolique ou par méthanisation.

**14.** Procédé de production de synthons et de biomolécules comprenant une étape de transformation, par voie biologique, d'une biomasse prétraitée selon la revendication 10.

**15.** Méthode de production de produits valorisables à partir d'une biomasse ligno-cellulosique comprenant :

- une étape de prétraitement de ladite biomasse par application du procédé de fractionnement selon l'une des revendications 1 à 9,
- optionnellement, une étape de transformation par voie biologique de ladite biomasse obtenue à l'étape précédente,
- une étape de récupération des produits valorisables obtenus.

Paille non traitée
2 mm

NaOH

H₂O₂

NH3

Imprégnation chimique basique : NaOH, H₂O₂, NH₃
Concentrations : 5 et 10% w/w biomasse

Humidité ≈ 30%

**Figure 1**

Figure 2

**Figure 3**

Figure 4

Figure 5

Figure 6

Figure 7

Figure 8

Figure 9

**Quantité de CH4 disponible après pré-traitement et méthanisation de la paille**

Figure 10

**Europäisches Patentamt**
**European Patent Office**
**Office européen des brevets**

# RAPPORT DE RECHERCHE EUROPEENNE

Numéro de la demande

EP 15 30 5091

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | BARAKAT ABDELLATIF ET AL: "Eco-friendly dry chemo-mechanical pretreatments of lignocellulosic biomass: Impact on energy and yield of the enzymatic hydrolysis", APPLIED ENERGY, vol. 113, 2014, pages 97-105, XP028762920, ISSN: 0306-2619, DOI: 10.1016/J.APENERGY.2013.07.015 * page 98, alinéa 2.1-2.3 * * page 99, ligne 2.5 * * page 100, alinéas 3,3.1 * ----- | 1-15 | INV. C10L3/08 C10L5/36 C10L9/08 |
| X | US 2015/007492 A1 (PIRIOU BRUNO [FR] ET AL) 8 janvier 2015 (2015-01-08) * alinéas [0056], [0080] - [0097] * ----- | 1-15 | |
| X | US 2012/060408 A1 (BARTEK ROBERT [US] ET AL) 15 mars 2012 (2012-03-15) * alinéas [0019] - [0031]; revendications 1-27 * ----- | 1-15 | |
| A | US 2014/338217 A1 (BJÖRKLUND PETER [SE]) 20 novembre 2014 (2014-11-20) * abrégé; revendications; figures * ----- | 1-15 | DOMAINES TECHNIQUES RECHERCHES (IPC) C10L |

Le présent rapport a été établi pour toutes les revendications

| Lieu de la recherche | Date d'achèvement de la recherche | Examinateur |
|---|---|---|
| La Haye | 11 juin 2015 | Bertin, Séverine |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
......................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 15 30 5091

11-06-2015

| Document brevet cité au rapport de recherche | Date de publication | Membre(s) de la famille de brevet(s) | Date de publication |
|---|---|---|---|
| US 2015007492 A1 | 08-01-2015 | AR 090413 A1<br>CA 2861243 A1<br>EP 2804932 A1<br>FR 2985735 A1<br>US 2015007492 A1<br>WO 2013108177 A1 | 12-11-2014<br>25-07-2013<br>26-11-2014<br>19-07-2013<br>08-01-2015<br>25-07-2013 |
| US 2012060408 A1 | 15-03-2012 | US 2012060408 A1<br>WO 2010068773 A1 | 15-03-2012<br>17-06-2010 |
| US 2014338217 A1 | 20-11-2014 | CA 2857246 A1<br>EP 2785817 A1<br>US 2014338217 A1<br>WO 2013081510 A1 | 06-06-2013<br>08-10-2014<br>20-11-2014<br>06-06-2013 |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **GIRIO et al.** *Biosource Technology,* 2010, vol. 101, 4775-4800
- **CHEW et al.** Renewable and Sustainable Energy. *Reviews,* 2011, vol. 15, 4212-4222
- **BARAKAT et al.** *Biosource Technology,* 2013, vol. 134, 362-373
- **VAN DYK et al.** *Biotechnology Advances,* 2012, vol. 30, 1458-1480

- **REPELLIN et al.** *Biomass and Energy,* 2010, vol. 34 (7), 923-930
- **CHEW et al.** *Renewable and Sustainable Energy Reviews,* 2011, vol. 15, 4212-4222
- **BARAKAT et al.** *Applied Energy,* 2014, vol. 113, 97-105